# EUROPEAN PATENT SPECIFICATION

(11) **EP 4 536 855 B1**
(45) Date of publication and mention of the grant of the patent: **31.12.2025**
(21) Application number: 23733040.2
(22) Date of filing: 08.06.2023
(51) Int. Cl.: C12Q 1/6806, C12N 15/10, C12N 15/00

(54) **NUCLEIC ACID AND GENE SYNTHESIS**
NUKLEINSÄURE- UND GENSYNTHESE
SYNTHÈSE D'ACIDES NUCLÉIQUES ET DE GÈNE

(30) Priority: 10.06.2022 GB 202208495
(43) Date of publication of application: 16.04.2025
(73) Proprietor: NunaBio Limited, Newcastle upon Tyne NE1 4BF (GB)
(72) Inventor: PIKE, Andrew Robert, Newcastle upon Tyne Tyne and Wear NE3 2HR (GB); TUITE, Eimer Mary, Newcastle upon Tyne Tyne and Wear NE7 7RZ (GB); HEDLEY, Joseph Henry, Longframlington Morpeth Northumberland NE65 8BQ (GB)
(74) Representative: Definition IP Limited
(86) International application number: PCT/GB2023/051497
(87) International publication number: WO 2023/237889

(56) References cited:
- WO-A1-2019/162699
- COLETTE J WHITFIELD ET AL: "Self-Priming Enzymatic Fabrication of Multiply Modified DNA", CHEMISTRY - A EUROPEAN JOURNAL, JOHN WILEY & SONS, INC, DE, vol. 24, no. 57, 14 September 2018 (2018-09-14), pages 15267 - 15274, XP071846733, ISSN: 0947-6539, DOI: 10.1002/CHEM.201801976
- COLETTE J WHITFIELD ET AL: "Enzymatic Method for the Synthesis of Long DNA Sequences with Multiple Repeat Units", ANGEWANDTE CHEMIE INTERNATIONAL EDITION, VERLAG CHEMIE, HOBOKEN, USA, vol. 54, no. 31, 10 June 2015 (2015-06-10), pages 8971 - 8974, XP072068332, ISSN: 1433-7851, DOI: 10.1002/ANIE.201502971
- HEMAT F ET AL: "A Rapid and Efficient PCR-Based Method for Synthesizing High-Molecular-Weight Multimers of Oligonucleotides", BIOCHEMICAL AND BIOPHYSICAL RESEARCH COMMUNICATIONS, ELSEVIER, AMSTERDAM NL, vol. 205, no. 1, 30 November 1994 (1994-11-30), pages 475 - 481, XP024765631, ISSN: 0006-291X, [retrieved on 19941130], DOI: 10.1006/BBRC.1994.2690
- JADHAV VASANT R. ET AL: "Design of a Combinatorial Oligonucleotide Library Containing All Possible Hexamer Palindromes: PCR Synthesis and Application for Identifying Restriction Cleavage Sites", BIOCHEMICAL AND BIOPHYSICAL RESEARCH COMMUNICATIONS, vol. 242, no. 2, 1 January 1998 (1998-01-01), Amsterdam NL, pages 297 - 302, XP093082051, ISSN: 0006-291X, DOI: 10.1006/bbrc.1997.7957

## Description

### Technical Field

The present invention relates to methods of synthesising nucleic acids. The present invention also relates to overlapping primer oligonucleotides for use in nucleic acid synthesis reactions.

### Background

Nucleic acid synthesis is the process whereby nucleotides are brought together to form nucleic acids such as deoxyribonucleic acid (DNA) and ribonucleic acid (RNA). Nucleic acids can be synthesised using a number of techniques such as phosphoramidite synthesis, the slippage reaction, loop-mediated amplification (LAMP), primer-template extension and thermal cycling amplification.

The synthesis of nucleic acids containing repeating sequences is challenging for the existing methods of nucleic acid synthesis, particularly in the synthesis of longer sequences (over 200 base pairs (bp)). Typically, the challenges associated with synthesising longer nucleic acid sequences are due to a reduction in yield and quality when producing longer sequences. One of the most commonly used methods of nucleic acid synthesis is column-based oligonucleotide synthesis which employs phosphoramidite synthesis chemistry to sequentially add bases to a growing oligonucleotide sequence on a solid support. This method utilises a four-step process of de-blocking (detritylation), coupling, capping and oxidation which is repeated to build up the oligonucleotide sequence. Once the synthesis is complete, the oligonucleotide is chemically cleaved from the solid support. This process is particularly amenable to automation and is therefore the basis of many commercial gene synthesiser systems. The major drawback with column-based oligonucleotide synthesis is that the yield and quality of the oligonucleotide products decrease with sequence length. This is due to spurious depurination, particularly on adenosines, during the synthesis. These spurious sites promote cleavage of the oligonucleotide backbone resulting in low yields. This reduction in yield and quality means that this method is not suitable for generation of oligonucleotides above 200 bases in length. Phosphoramidite synthesis is also inefficient in the production of sequences having a high GC content, hairpin structures and/or highly repetitive sequences.

Alternatively, enzymatic techniques can be used to synthesise nucleic acids. Typically, these methods have fewer restrictions on sequence length when compared to phoshoramidite synthesis methods. Enzymatic techniques utilise a nucleic acid template or primer and polymerase enzyme to synthesise nucleic acid sequences using the polymerase chain reaction (PCR). One such example is polymerase cycling assembly (PCA). PCA uses PCR to extend short overlapping oligonucleotides into a double stranded sequence. Although enzymatic synthesis techniques are suitable for the generation of longer sequences, they are inefficient in the production of highly repetitive sequences and sequences with high GC content.

WO2019162699 describes a thermocycling based method that can be used to increase the number of tandem repeats of a unit sequence.

Colette J Whitfield et al., Angew. Chem. Int. Ed. (2015) 8971-8974 describes an enzymatic method for the synthesis of long DNA sequences with multiple repeat units, wherein the method is in solution. Colette J Whitfield et al., Chem. Eur. J. 24 (2018) 15267-15274 further describes the use of this method for self-priming synthesis of modified DNA.

Hemat F et al., Biochem. Biophys. Res. Commun. (1994) 475-481 describes a PCR-based method, performed in solution, for synthesizing high molecular weight multimers of oligonucleotides.

Jadhav Vasant R et al., Biochem. Biophys. Res. Commun. (1998) 297-302 describes an algorithm for designing a combinatorial library representing hexamer palindromes.

Long nucleic acid sequences comprising multiple repeat units have many uses in synthetic biology, diagnostics and therapeutics and therefore there is a need to identify a more efficient method for their production.

### Summary of the Invention

According to a first aspect of the present invention, there is provided a method for extending the length of a nucleic acid, the method comprising the steps of;
i) providing at least one overlapping primer oligonucleotide, said overlapping primer oligonucleotide comprising a first single-stranded oligonucleotide and a second single-stranded oligonucleotide, each of the first and second single-stranded oligonucleotides comprising at least a first, second and third sequence, said third sequence being located at the 3' end of each single-stranded oligonucleotide and said second sequence being located between the first and third sequences; wherein the first sequences and the third sequences of the first single-stranded oligonucleotide and the second single-stranded oligonucleotide are self-complementary, palindromic sequences;
   wherein the third sequence of the first single-stranded oligonucleotide hybridises to the third sequence of the second single-stranded oligonucleotide to provide an overhanging region at the 5' end of at least one of the first or second single-stranded oligonucleotides;
   and wherein the second sequence on the first single-stranded oligonucleotide differs from the second sequence on the second single-stranded oligonucleotide;
(ii) enzymatically extending both the first and second single-stranded oligonucleotides to provide a duplex.

Advantageously, extension step (ii) incorporates a fourth sequence into the first single-stranded oligonucleotide, said fourth sequence being complementary to the second sequence on the second single-stranded oligonucleotide and wherein a fourth sequence is incorporated into the second single-stranded oligonucleotide, said fourth sequence being complementary to the second sequence on the first single-stranded oligonucleotide.

Advantageously, the present invention provides an improved method of synthesising nucleic acids. In particular, this method provides a more efficient means to produce longer sequences of nucleic acids which can be designed in accordance with the users requirements.

Furthermore, the method provides an improved means of synthesising long repeating sequences which are difficult to synthesise using existing methods due to low yields and high error rates.

Advantageously, having a 3' self-complementary sequence ensures the predictable incorporation of sequences into the oligonucleotides during extension permitting the user more control over the ordering of the sequences in the nucleotide product.

Typically, a fourth sequence is incorporated into the first and second single-stranded oligonucleotides during step (ii), said fourth sequence on the first single-stranded oligonucleotide being complementary to the second sequence on the second single-stranded oligonucleotide and said fourth sequence on the second single-stranded oligonucleotide being complementary to the second sequence on the first single-stranded oligonucleotide.

Optionally, the overlapping primer oligonucleotide is a DNA oligonucleotide.

Optionally, overlapping primer oligonucleotide is an RNA oligonucleotide.

Advantageously, when the second sequence differs between the first single-stranded oligonucleotide and the second-single stranded oligonucleotide, a greater variety of sequences can be incorporated into the extending oligonucleotides due to complementary base pairing resulting in two distinct fourth sequences being incorporated into the nucleic acid sequence.

Preferably, the first and third sequences are each at least 6 bases in length and are preferably at least 8 bases in length.

Preferably, the second and fourth sequences are each at least 2 bases in length.

Optionally, at least one of the first and third sequences comprises at least one functional site.

Optionally the at least one functional site is in addition to the self-complementary, palindromic sequences (and could be considered separate sequences to the self-complementary, palindromic first and third sequences).

Optionally the at least one functional site is itself a self-complementary, palindromic sequence.

Optionally, the at least one functional site is a restriction enzyme site and/or a capping site.

Optionally, the at least one functional site is selected from the following: restriction enzyme site, capping site, chemically reactive modifications to the bases, e.g. alkynes, azides, halo-modifications, sequence specific drug binding site, enzyme inhibitor site.

In terms of a functional site that is a chemically reactive modification to the bases, the at least one functional site is selected from the following: alkyne-modifications, azide-modifications, halo-modifications.

In terms of a functional site that is more than one modified base, the at least one functional site is selected from the following: restriction enzyme site, capping site, sequence specific drug binding sites, enzyme inhibitor sites etc.

Advantageously, the inclusion of a restriction site in one of the sequences provides a means by which the nucleic acid sequence can be readily cut into shorter sequences. For example, sequences A and C may encode two desired oligonucleotides and sequences B and D may encode two restriction enzyme binding sites. In this example, once a long repeating sequence is generated in accordance with the method described herein, the relevant restriction enzymes can be used to readily digest the long repeating sequence into the desired oligonucleotide sequences.

Optionally, at least one of the first and third sequences comprise tandem repeats.

Optionally, both the first and third sequences comprise of tandem repeats.

Optionally, the tandem repeat is a di-nucleotide tandem repeat.

Advantageously, when the first and third sequences comprise di-nucleotide tandem repeats, controlled incorporation of modified bases into the second and fourth sequences is possible.

Optionally, at least one of the second and fourth sequences comprises at least one modification selected from the following: modified nucleotides, artificial bases, loop structures.

Advantageously, modified nucleotides can be readily incorporated into nucleic acid sequences using the method of the present invention. Modified nucleotides are used to alter the characteristic of nucleic acids which is particularly desirable in fields such as nanotechnology, biomedicine and diagnostics.

Optionally, the modified nucleotides are alkyne, azide or phosphorothioate modified nucleotides.

Optionally, the modified nucleotides may comprise: 5-Br-dUTP, 7-deaza-7-I-dATP, 6-S-dGTP,5-I-dCTP, 5-(octadiynyl)-dCTP, dye-labelled nucleotides, quencher-labelled nucleotides, intrinsically fluorescent nucleotides, α-phosphate modified nucleotides, γ-[(6-aminohexyl)-imido]-ATPαS, α,β non-hydrolyzable nucleotides, β-Phosphate modified nucleotides, β,γ non-hydrolyzable nucleotides, γ-Phosphate modified nucleotides, non-hydrolyzable di-nucleotides, non-hydrolyzable dye-labeled nucleotides, biotin labelled nucleotides, desthiobiotin labelled nucleotides, digoxigenin labelled nucleotides, DNP (dinitrophenol) labelled nucleotides, photo-labile groups labelled nucleotides, DBCO labelled nucleotides, TCO labelled nucleotides, vinyl labelled nucleotides, free amino group (-NH2) labelled nucleotides, redox dye labelled nucleotides, halogen atom labelled nucleotides, mercury labelled nucleotides, selenium labelled nucleotides, ferrocence labelled nucleotides, analogs and derivatives of cap, analogs and derivatives of puromycin, analogs and derivatives of coenzyme A (CoA), analogs and derivatives of NAD, analogs and derivatives of natural RNA nucleobases, cyclic-di-nucleotides, 3',5'-cyclic nucleotides, 2',3'-cyclic nucleotides, dinucleoside polyphosphates, 6-thio purines, 7-deaza purines, 7-methyl guanosines, substituted pyrimidines, 5-methyl cytidines and related, unmodified and modified ddNTPs 2'-Fluoro-2'-NTPs, 2'-O-Methyl-NTPs, LNA-NTPs, cleavable base labelled dNTP's, N1-modified purines, N6-modified purines, 6-modified purines, 8-oxo guanosines, 2'-deoxy uridines, 3'-deoxy nucleotides, nucleoside bisphosphates, ara-nucleotides, unmodified Purines, modified dNTPs, 3'-O-azidomethyl-dNTPs.

Optionally, the modified nucleotides comprise a linker which attaches the modification to the nucleotide.

Advantageously, using a linker to attach the modification to the base prevents the modification interfering with base recognition sites.

Optionally, the loop structure is a G quadruplex region or a C-motif or intercalated-motif (i-motif) DNA.

Preferably, the method further comprises the steps:
(iii) denaturing the duplex to provide a first and second single-stranded polynucleotide;
(iv) annealing the first and second single-stranded polynucleotides such that overhanging region is provided at the 5' end of at least one of the first or second single-stranded polynucleotides to permit polynucleotide duplex formation between the first and second single-stranded polynucleotides;
(v) enzymatically extending both the first and second single-stranded polynucleotides to provide a duplex.

Preferably, steps (iii) to (v) are repeated to increase the length of the nucleic acid sequence.

Optionally, the overlapping primer oligonucleotide is immobilised on a surface.

Preferably, the overlapping primer oligonucleotide is immobilised on a surface by the 5' end of one of the first or second single-stranded polynucleotides.

Preferably, the surface comprises glass, silica, gold, graphene, graphene oxide, epoxy, plastic, metal, gel matrix, template stripped metals or composites thereof.

Optionally, wherein the overlapping primer oligonucleotide is immobilised to the surface by covalent or non-covalent bonding.

Optionally, the overlapping primer oligonucleotide is immobilised to a chemically modified region of the surface.

Optionally, the overlapping primer oligonucleotide is immobilised to the surface by a linker.

Optionally, the linker comprises a silane linker molecule, a biotin-streptavidin complex, a thiol-Au linker, covalent Si-C bonds to silicon, covalent Si-O bonds to silicon, covalent Si-N bonds to silicon, a nanoparticle linker, or a dynamic covalent bond.

Preferably the step of providing at least one overlapping primer oligonucleotide comprises obtaining a first single-stranded oligonucleotide and second single-stranded oligonucleotide and hybridising the first and second single-stranded oligonucleotides under appropriate conditions.

According to a second aspect of the present invention, there is provided an overlapping primer oligonucleotide for extending the length of repeating sequences of nucleic acid, said overlapping primer oligonucleotide comprising two partially complementary single-stranded oligonucleotides, each single-stranded oligonucleotide comprising at least a first, second and third sequence wherein the third sequence is located at the 3' end of the first and second single-stranded oligonucleotide; the first sequences and the third sequences are palindromic, self-complementary sequences; and the second sequence is located in between the first and third sequences; wherein the third sequence of the first single-stranded oligonucleotide hybridises to the third sequence of the second single-stranded oligonucleotide to provide an overhanging region at the 5' end of at least one of the first or second single-stranded oligonucleotides; and wherein the second sequence on the first single-stranded oligonucleotide differs from the second sequence on the second single-stranded oligonucleotide and/or wherein the second sequence on the first single-stranded oligonucleotide is not complementary to the second sequence on the second single-stranded oligonucleotide.

Preferably, the first and third sequences are each at least 6 bases in length and are preferably at least 8 bases in length.

Preferably, the second sequence is at least 2 bases in length.

Optionally, at least one of the first and third sequences comprises at least one functional site.

Optionally, the at least one functional site is selected from the following: restriction enzyme site, capping site.

Optionally, at least one of the first and third sequences comprise tandem repeats.

Optionally, both the first and third sequences comprise tandem repeats.

Optionally, the tandem repeat is a dinucleotide tandem repeat.

Optionally, the second sequence comprises at least one modification selected from the following: modified nucleotides, artificial bases, loop structures.

Optionally, the modified nucleotides are alkyne, azide or phosphorothioate modified nucleotides. Optionally, these may include: 5-Br-dUTP, 7-deaza-7-I-dATP, 6-S-dGTP,5-I-dCTP, 5-(octadiynyl)-dCTP.

Optionally, the modified nucleotides comprise a linker which attaches the modification to the nucleotide.

Optionally, the loop structure is a G quadruplex region.

Optionally, the overlapping primer oligonucleotide is a DNA primer oligonucleotide.

Optionally, overlapping primer oligonucleotide is an RNA primer oligonucleotide.

Various further features and aspects of the invention are defined in the claims.

Unless defined otherwise, all technical and scientific terms used herein have the meaning commonly understood by a person skilled in the art to which this invention belongs.

A 'palindromic sequence' is a nucleic acid sequence in a double-stranded DNA or RNA molecule whereby reading in a certain direction (e.g. 5' to 3') on one strand is identical to the sequence in the same direction (e.g. 5' to 3') on the complementary strand.

A 'duplex' is a double stranded nucleic acid sequence comprising two complementary sequences annealed to one another. A "partial duplex" is a double stranded nucleic acid sequence wherein a section of one of the strands is complementary to the other strand and anneals to form a partial duplex, but the full lengths of the strands are not complementary, resulting in a single-stranded polynucleotide tail at least at one end of the partial duplex.

The terms 'hybridisation' 'binding' and "annealing" (or 'hybridise', 'bind' and 'anneal') in the context of nucleotide sequences, are used interchangeably herein. The ability of two nucleotide sequences to hybridize with each other is based on the degree of complementarity of the two nucleotide sequences, which in turn is based on the fraction of matched complementary nucleotide pairs. The more nucleotides in a given sequence that are complementary to another sequence, the more stringent the conditions can be for hybridization and the more specific the binding of the two sequences will be. Increased stringency is typically achieved by elevating the temperature, increasing the ratio of cosolvents, lowering the salt concentration, and other such methods well known in the field.

The term "hybridisation conditions" refers to the reagents and reaction conditions (e.g. temperature, time etc) that are used for hybridisation. Typically, hybridisation conditions may be stringent or moderate. The hybridisation conditions used in the context of the methods described herein permit mismatched duplex formation and therefore may be either moderate or stringent. Preferably, the hybridisation between the unit sequence of the first single-stranded oligonucleotide and a second single-stranded oligonucleotide will form a stable duplex at 65° C. and below. It is preferred that a mismatched duplex may be formed at temperatures up to 65° C., for example between 55° C. and 65° C., optionally for a time period of between 1 to 30 seconds.

Moderate and stringent conditions are known to those skilled in the art and can be found in available references (e.g., Current Protocols in Molecular Biology, John Wiley & Sons, N.Y., 1989, 6.3.1-6.3.6). Aqueous and non-aqueous methods are described in that reference and either can be used. A preferred example of stringent hybridization conditions are hybridization in 6× sodium chloride/sodium citrate (SSC) at about 45° C., followed by one or more washes in 0.2×SSC, 0.1% (w/v) SDS at 50° C. Another example of stringent hybridization conditions are hybridization in 6×SSC at about 45° C., followed by one or more washes in 0.2×SSC, 0.1% (w/v) SDS at 55° C. A further example of stringent hybridization conditions are hybridization in 6×SSC at about 45° C., followed by one or more washes in 0.2×SSC, 0.1% (w/v) SDS at 60° C. Preferably, stringent hybridization conditions are hybridization in 6×SSC at about 45° C., followed by one or more washes in 0.2×SSC, 0.1% (w/v) SDS at 65° C. Particularly preferred stringency conditions (and the conditions that should be used if the practitioner is uncertain about what conditions should be applied to determine if a molecule is within a hybridization limitation of the invention) are 0.5 molar sodium phosphate, 7% (w/v) SDS at 65° C., followed by one or more washes at 0.2×SSC, 1% (w/v) SDS at 65° C.

The terms 'extension' or 'elongation' in the context of nucleotide sequences, are used interchangeably herein. They refer to the extension of a 3'-end and/or a 5' end of a polynucleotide by the addition of nucleotides or bases. Chain extension relevant to the present invention is generally template dependent, that is, the appended nucleotides are determined by the sequence of a template nucleic acid to which the extending chain is hybridised.

The term 'enzymatic extension' refers to the extension of a nucleic acid which is catalysed by an enzyme, such as a polymerase enzyme. It is preferred that the polymerase enzyme can be template dependent, such as Deep Vent^{®} polymerase.

The term "extension conditions" refers to the reagents and reaction conditions (e.g. temperature, time etc) that are used. It describes conditions for extension of the primer polynucleotide. In the present invention, contact between the mismatched duplex, polymerase and nucleotides is under extension conditions that permit polynucleotide extension in a 5' to 3' direction. Appropriate extension conditions are well known in the art. Preferably, extension is performed at a temperature of between about 65° C. and 75° C., optionally for a time period of between 30 to 120 seconds. Appropriate conditions may be found, for example, in Whitfield C J, Turley A T, Tuite E M, Connolly B A, Pike A R. Enzymatic Method for the Synthesis of Long DNA Sequences with Multiple Repeat Units. Angewandte Chemie International Edition 2015, 54(31), 8971-8974.

The term 'complementary' in the context of nucleotide sequences refers to when the sequence of one can bind to the sequence of the other in an anti-parallel sense wherein the 3'-end of one sequence binds to the 5'-end of the other sequence and each A, T(U), G, and C of one sequence is then aligned with a T(U), A, C, and G, respectively, of the other sequence.

The term 'amplification', as applied to nucleic acids refers to any method that results in the formation of one or more copies of a nucleic acid, where preferably the amplification is exponential. One such method for enzymatic amplification of specific sequences of DNA is known as the polymerase chain reaction (PCR), as described by Saiki et al., 1986, Science 230:1350-1354.

The terms 'nucleic acid' and 'polynucleotide' are interchangeable and refer to any nucleic acid, whether DNA, RNA, cDNA, DNA-RNA, peptide nucleic acid (PNA), a hybrid or any mixture of the same.

The terms 'nucleic acid', 'polynucleotide' and 'nucleotide' also specifically include nucleic acids composed of synthetic bases (i.e. bases other than the five biologically occurring bases - adenine, guanine, thymine, cytosine and uracil), modified bases or any combination of biologically occurring bases, synthetic bases and modified bases.

The term 'SS oligonucleotide' refers to 'single stranded oligonucleotide'.

The polynucleotides of the present invention can be from a human or non-human mammal, or any other organism, derived from any recombinant source, synthesized in vitro or by chemical synthesis.

### Brief Description of the Drawings

Embodiments of the present invention will now be described by way of example only with reference to the accompanying drawings where like parts are provided with corresponding reference numerals and in which:
Figure 1 provides a schematic representation of the method according to the present invention when the first sequence B on the first single-stranded oligonucleotide differs from the second sequence B on the second single-stranded oligonucleotide.
Figure 2 shows ultraviolet absorption of the PCR product after an increasing number of PCR cycles with the double-stranded primer oligonucleotide of example 1.
Figure 3A shows a visualisation of gel electrophoresis of the PCR products after 3, 4, 10 and 20 PCR cycles with the double-stranded primer oligonucleotide of example 1. Figure 3B shows the fluorescence intensity of the gel electrophoresis shown in figure 3A.
Figure 4 shows ultraviolet absorption of the PCR product after an increasing number of PCR cycles with the double-stranded primer oligonucleotide of example 2.
Figure 5A shows a visualisation of gel electrophoresis of the PCR products after 3, 4, 10 and 20 PCR cycles with the double-stranded primer oligonucleotide of example 2. Figure 5B shows the fluorescence intensity of the gel electrophoresis shown in figure 5A.
Figure 6 shows the double-stranded primer oligonucleotide of example 2 and the extended polynucleotide product.
Figure 7 shows ultraviolet absorption of the PCR product after an increasing number of PCR cycles with the double-stranded primer oligonucleotide of example 3.
Figure 8A shows a visualisation of gel electrophoresis of the PCR products after 3, 4, 10 and 20 PCR cycles with the double-stranded primer oligonucleotide of example 3. Figure 8B shows the fluorescence intensity of the gel electrophoresis shown in figure 8A.
Figure 9 shows the UV/vis spectra of the extended DNA following purification.
Figure 10 (A) shows a visualisation of gel electrophoresis of the extended DNA against the Gene Ruler 1kb plus DNA ladder, the fluorescence intensity of the gel electrophoresis was measured and analysed using Image-J software.
Figure 10 (B) shows a visualisation of gel electrophoresis of the digested products against a low range ladder, the fluorescence intensity of the gel electrophoresis was measured and analysed using Image-J software, the results are shown.

### Detailed Description

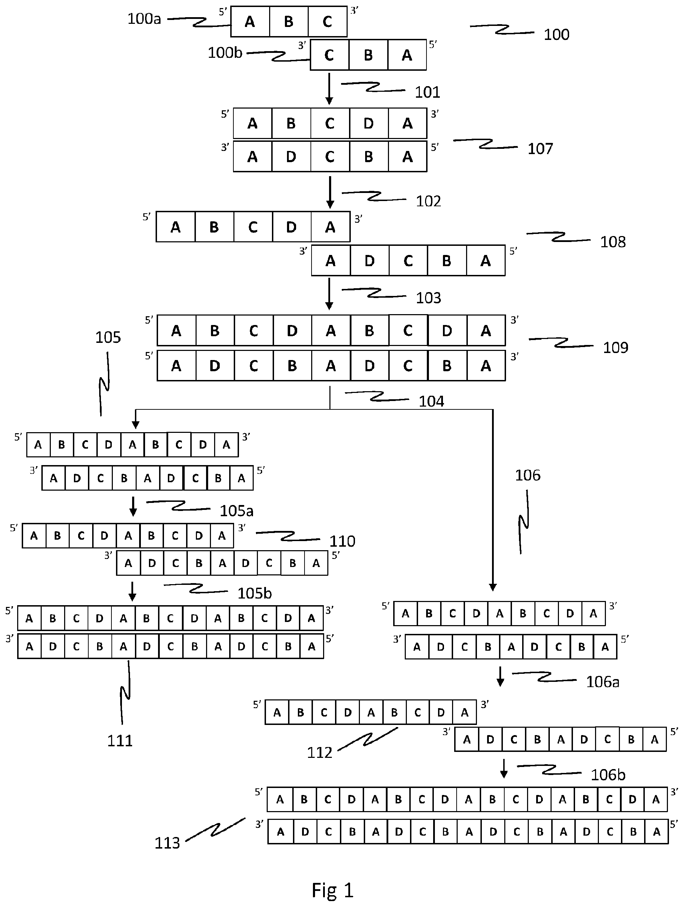

Figure 1 depicts a schematic representation of the method of the present invention. The starting overlapping primer oligonucleotide 100 comprises two single-stranded (SS) oligonucleotides 100a, 100b. Each of the SS oligonucleotides 100a, 100b comprises sequences A, B and C. The first SS oligonucleotide 100a comprises sequences A, B and C in a 5' to 3' direction. The second SS oligonucleotide 100b comprises sequences C, B and A in a 3' to 5' direction.

In this embodiment, the A sequences are palindromic, self-complementary sequences and the C sequences are palindromic, self-complementary sequences and all sequences are identical in each of the SS oligonucleotides. Sequence B is complementary to a fourth sequence, sequence D, which will be incorporated into the SS oligonucleotides by complementary base pairing as described below. Sequences A and C are at least 6, preferably 8 bases in length. Sequences B and D are at least 2 bases in length.

It would be apparent to the skilled person that, as required by the claimed invention, the nucleotide sequences of sequences B and D could differ between the first and second SS oligonucleotides provided that sequence B of the first SS oligonucleotide is complementary to sequence D of the second SS oligonucleotide and that sequence B of the second SS oligonucleotide is complementary to sequence D of the first SS oligonucleotide.

The overlapping primer oligonucleotide 100 is formed by the hybridisation of sequence C of the first SS oligonucleotide 100a with sequence C of the second SS oligonucleotide 100b. This hybridisation results in a partial duplex with an overhanging region on the 5' end of each of the SS oligonucleotide sequences 100a, 100b. The overhanging regions of each SS oligonucleotide comprise both sequence A and B.

The overlapping primer oligonucleotide 100 is extended 101 in the presence of a suitable polymerase and nucleotides under extension conditions. Any suitable polymerase can be used, for example Deep Vent DNA polymerase (New England Biolabs). Several well-known thermostable 5' to 3' polymerases are available and may be used in the methods described herein. It is preferred that a polymerase is thermostable and highly stable, such that its activity is substantially retained during prolonged incubation necessary for the extension reaction. The polymerase preferably has high processivity. It is preferred that a polymerase does not display non-specific nuclease activity. A polymerase preferably has good fidelity but can also accept a range of nucleotide analogues as both templates and substrates. A high-fidelity polymerase with highly efficient proof-reading activity is not particularly suitable. Preferably, a polymerase lacks 3'→5' exonuclease activity [3'→5' exo(-)], thereby possessing lower fidelity due to absence of proof-reading function. A person skilled in the art can determine whether a particular polymerase possesses required properties as defined above. Exemplary polymerases include, but are not limited to, Thermococcus gorgonarius family B polymerase (Tgo-Pol) enzyme variant, Z3 (Tgo-Pol Z3) exo(-) [Jozwiakowski et al., 2011 Chembiochem 12: 35-37], Deep Vent exo(-) (New England Biolabs), Vent exo(-) (New England Biolabs), Pfu exo(-) (Agilent Technologies), and Taq polymerase (many suppliers).

The extension of the overlapping primer oligonucleotides can be carried out using the PCR reaction. This results in the extension of the SS oligonucleotides 100a, 100b in the overhang regions to create a stable duplex 107. In this step, a fourth sequence (D) is incorporated into the SS oligonucleotides 100a, 100b. Sequence D is the complementary sequence to sequence B.

In order to further extend the sequences, the stable duplex 107 is denatured 102 and reannealed under suitable conditions resulting in a second partial duplex 108. The second partial duplex 108 is formed by the hybridisation of sequence A of the first SS oligonucleotide 100a with sequence A of the second SS oligonucleotide 100b. This hybridisation results in mismatched duplex with an overhanging region on the 5' end of each of the SS oligonucleotide sequences 100a, 100b. The overhanging regions on each SS oligonucleotide comprise sequences B, C and D once and sequence A twice.

In some embodiments the sequences are designed to have different melting temperatures (Tms) which allows the amount of mismatch to be controlled, however when Tms are similar then it is expected that 50% will form a mismatched duplex that can be extended.

The second partial duplex 108 is extended 103 in the presence of a suitable polymerase and nucleotides under extension conditions. This results in the extension of the SS oligonucleotides 100a, 100b in the overhanging regions to create a second stable duplex 109. Further sequences A, B, C and D are incorporated into each of the SS oligonucleotides 100a, 100b such that sequences B, C and D appear in each SS oligonucleotide twice and sequence A appears three times.

To further extend the SS oligonucleotides 100a, 100b the second stable duplex 109 is denatured 104. The further extension may occur under maximum overlap conditions 105 or minimum overlap conditions 106. Under maximum overlap conditions 105, the SS oligonucleotides anneal 105a such that there is a maximum amount of overlap between the first and second SS oligonucleotides 100a, 100b. In maximum overlap conditions 105, a third partial duplex 110 is formed by the hybridisation of sequences A, B, C, D and A of the first SS oligonucleotide 100a with sequences A, D, C, B and A of the second SS oligonucleotide 100b. This hybridisation results in a third mismatched duplex 110 with an overhanging region on the 5' end of each of the SS oligonucleotide sequences 100a, 100b. The overhanging regions on each SS oligonucleotide comprise sequences A, B, C and D.

The reaction can be directed towards minimum overlap conditions by altering the temperatures of the denaturing and/or annealing steps. For example, increasing the denaturing and annealing temperatures can direct the reaction to minimum overlap conditions. It is also possible that, after a few cycles of extension reactions, increasing only the annealing temperature can direct the reaction to minimum overlap conditions. Directing the reaction to minimum overlap conditions is advantageous as it leads to products which are on average longer.

The third partial duplex 110 is extended 105b in the presence of a suitable polymerase and nucleotides under extension conditions. This results in the extension of the SS oligonucleotides 100a, 100b in the overhang regions to create a third stable duplex 111. Further sequences A, B, C and D are incorporated into each of the SS oligonucleotides 100a, 100b such that each SS oligonucleotide contains sequences B, C and D three times and sequence A four times.

Under minimum overlap conditions 106, the SS oligonucleotides anneal 106a such that there is a minimum amount of overlap between the first and second SS oligonucleotides 100a, 100b. In minimum overlap conditions 106, a fourth partial duplex 112 is formed by the hybridisation of sequence A of the first SS oligonucleotide 100a with sequence A of the second SS oligonucleotide 100b. This hybridisation results in a fourth partial duplex 112 with an overhanging region on the 5' end of each of the SS oligonucleotide sequences 100a, 100b. The overhanging regions on each SS oligonucleotide comprise sequences A, B, C and D twice.

The fourth partial duplex 112 is extended 106b in the presence of a suitable polymerase and nucleotides under extension conditions. This results in the extension of the SS oligonucleotides 100a, 100b in the overhanging regions to create a fourth stable duplex 113. Two further of sequences A, B, C and D are incorporated into each of the SS oligonucleotides 100a, 100b such that each SS oligonucleotide now contains sequences B, C and D four times and sequence A 5 times.

The denaturing 104, annealing 105a, 106a and extension steps 105b, 106b can be repeated any number of times to continue extending the sequences.

### Example 1: ^{5'}-(AT)₅C₂(TA)₅-^{3'} / ^{3'}-(AT)₅C₂(TA)₅-^{5'}

In one example of the present invention, the starting overlapping primer oligonucleotide comprised a first SS oligonucleotide and a second SS oligonucleotide. All custom oligonucleotides were purchased from Eurofins (Ebersberg, Germany). Each of the first and second SS oligonucleotides comprised identical sequences A, B and C and as such this example is not according to the invention and is present for illustration purposes only. The nucleotide sequences of sequences A, B and C are shown in Table 1.

**Table 1: Example 1 nucleotide sequences**

| **Sequence name** | **Nucleotide sequence** | |
|---|---|---|
| Sequence A | ATATATATAT | SEQ ID 1 |
| Sequence B | CC | SEQ ID 2 |
| Sequence C | TATATATATA | SEQ ID 3 |
| Sequence D | GG | SEQ ID 4 |

The first SS oligonucleotide comprises the sequences in the following order: ⁵' - ABC - ³' (SEQ ID 5) and the second SS oligonucleotide comprises the sequences in the following order ³' - CBA - ⁵' (SEQ ID 6).

Sequences A and C are palindromic, self-complementary sequences and therefore the first and second SS oligonucleotides hybridise to form the overlapping primer oligonucleotide, which is a duplex with 5' overhanging regions containing sequences A and B as shown in figure 1 (100). The overlapping primer oligonucleotide length is 22 base pairs. Sequence D is complementary to sequence B and will be incorporated into the oligonucleotides during the enzymatic extension. The nucleotide sequence of sequence D is shown in Table 1 above.

In the present example, sequences A and C comprise dinucleotide repeats of bases A and T. When sequences A and C are palindromic, self-complementary sequences and contain only 2 of the 4 DNA nucleotide bases (in this example A and T), modified bases can be incorporated into sequences B and D in a controlled manner. Advantageously, this allows the user greater control over where the modified bases incorporate and therefore greater control over the composition and characteristics of the extended polynucleotide product. The skilled person would understand that this is only one example of the present invention and in other examples, sequences A and C may contain sequences other than dinucleotide repeats. Likewise, it would be understood by the skilled person that in other examples sequences B and D may contain sequences other than mononucleotide repeats.

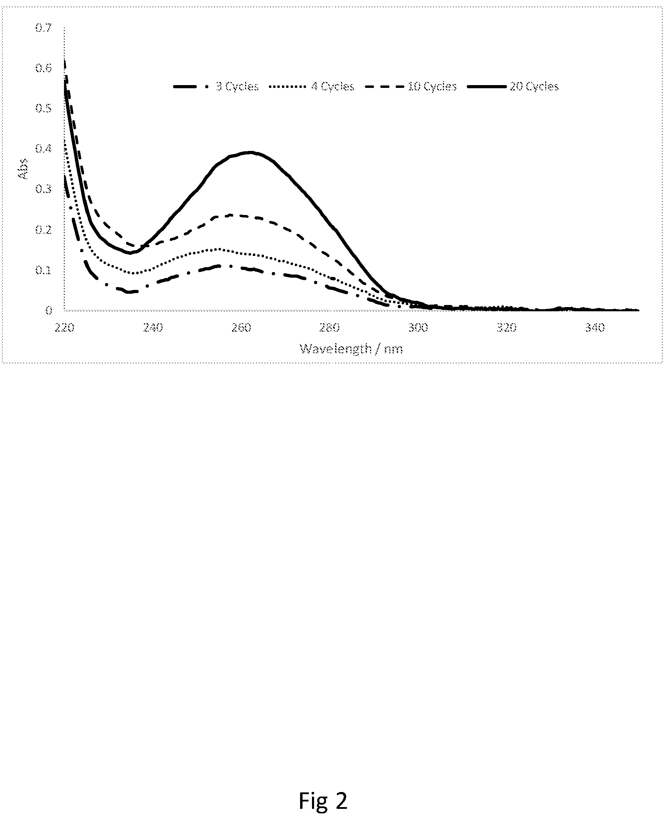

Figure 2 shows ultraviolet absorption of the PCR product after an increasing number of PCR cycles. Increased absorption of UV light at a wavelength of 260 nm indicates increased DNA concentration. Figure 2 shows that the concentration of DNA increases with an increased number of PCR cycles, demonstrating generation of new DNA with increasing PCR cycle numbers.

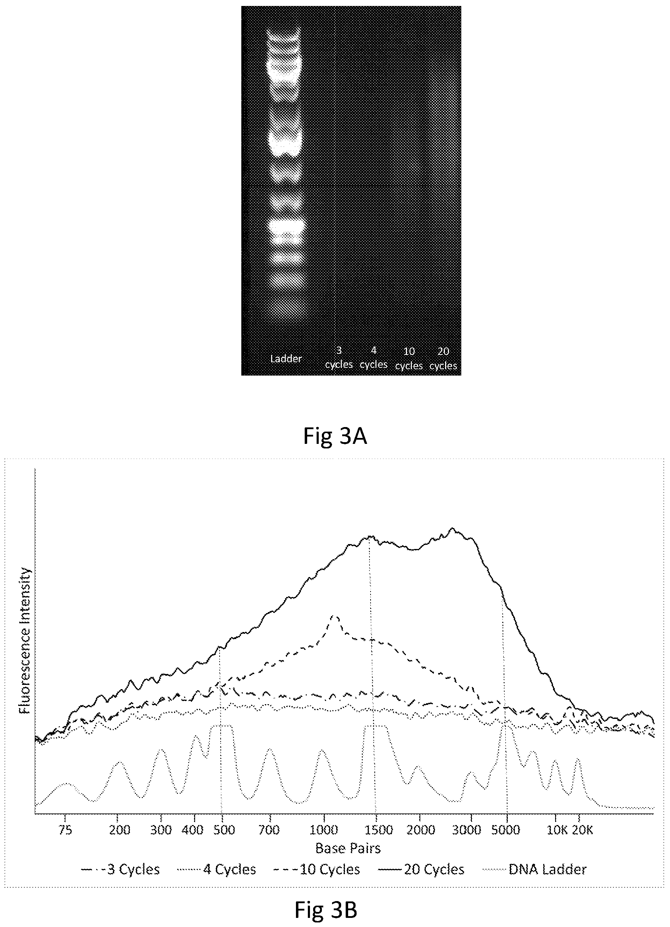

Figure 3A shows a visualisation of gel electrophoresis of the PCR products after 3, 4, 10 and 20 PCR cycles. The fluorescence intensity of the gel electrophoresis was measured and analysed using Image-J software, the results are shown in figure 3B. As can be seen in figure 3B, after 20 PCR cycles DNA products can be detected of between 1000 and 5000 base pairs in length.

### Example 2: ^{5'}-(AT)₅C₄(TA)₅-^{3'} / ^{3'}-(AT)₅C₄(TA)₅-^{5'}

In another example of the present invention, the starting overlapping primer oligonucleotide comprised a first and second SS oligonucleotide. Each of the first and second SS oligonucleotides comprised identical sequences A, B and C and as such this example is not according to the invention and is present for illustration purposes only. The nucleotide sequences of sequences A, B and C are shown in Table 2.

**Table 2: Example 2 nucleotide sequences**

| **Sequence name** | **Nucleotide sequence** | |
|---|---|---|
| Sequence A | ATATATATAT | SEQ ID 7 |
| Sequence B | CCCC | SEQ ID 8 |
| Sequence C | TATATATATA | SEQ ID 9 |
| Sequence D | GGGG | SEQ ID 10 |

The first SS oligonucleotide comprises the sequences in the following order: ^{5'} - ABC - ^{3'} (SEQ ID 11) and the second SS oligonucleotide comprises the sequences in the following order ³' - CBA - ^{5'} (SEQ ID 12).

Sequences A and C are palindromic, self-complementary sequences and therefore the first and second SS oligonucleotides hybridise to form a duplex with 5' overhang containing sequences A and B as shown in figure 1 (100). Sequence D is complementary to sequence B and will be incorporated into the oligonucleotides during the enzymatic extension. The nucleotide sequence of sequence D is shown in Table 2 above.

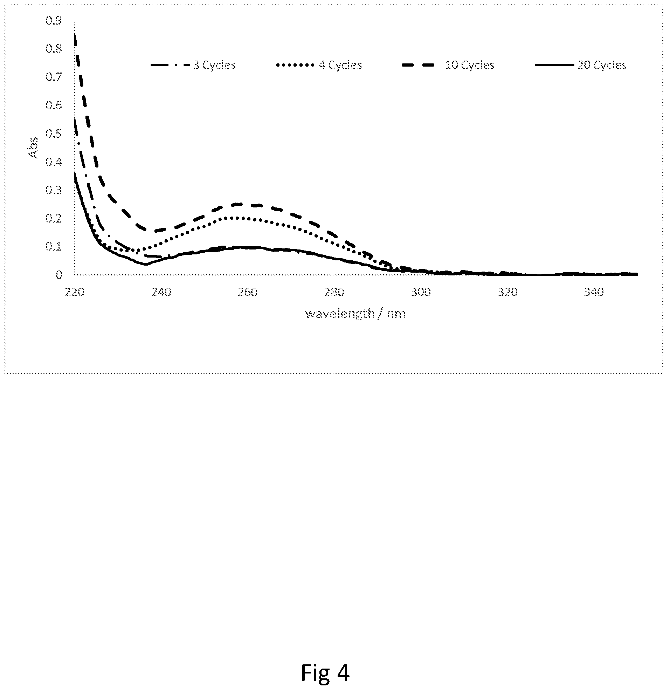

UV absorbance was measured as described in example 1. Figure 4 shows the UV absorbance data. Increased absorption of UV light at a wavelength of 260 nm indicates increased DNA concentration. As can be seen in figure 4, the DNA concentration in the PCR product increases with an increased number of PCR cycles, demonstrating generation of new DNA with increasing PCR cycle numbers.

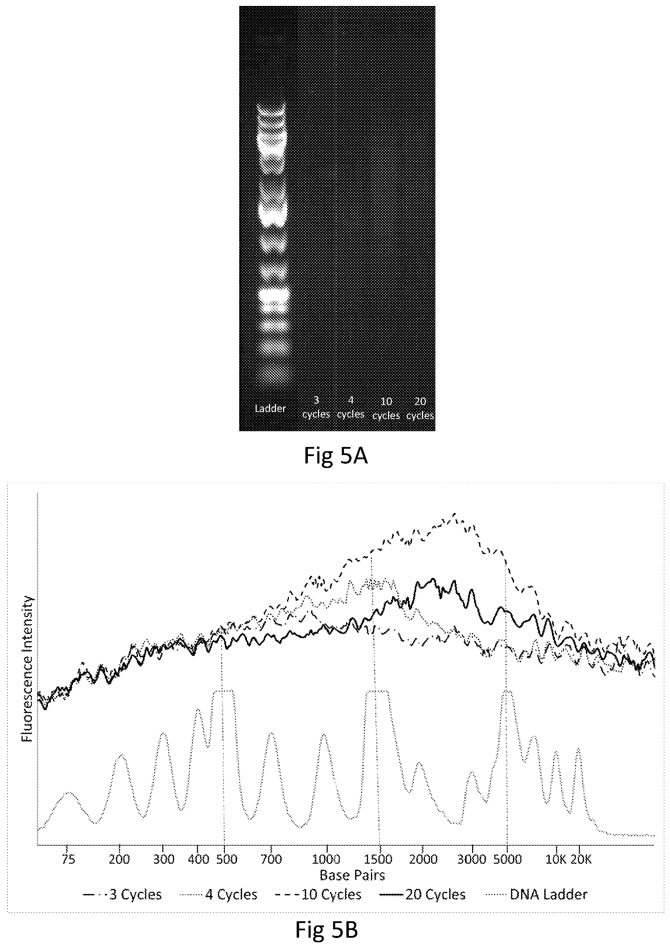

Figure 5A shows a visualisation of gel electrophoresis of the PCR products after 3, 4, 10 and 20 PCR cycles. The fluorescence intensity of the gel electrophoresis was measured and analysed using Image-J software, the results are shown in figure 5B. As can be seen in figure 5B, after 20 PCR cycles DNA products can be detected of between 1000 and 5000 base pairs in length.

### Example 3: ^{5'}-(AT)₅C₂(TA)₅-^{3'} / ^{3'}-(AT)₅C₈(TA)₅-^{5'}

In another example of the present invention, the starting overlapping primer oligonucleotide comprised a first and second SS oligonucleotide.

The first SS oligonucleotide comprised sequence A, sequence B and sequence C. The second SS oligonucleotide comprised sequence A, sequence B' and sequence C. The nucleotide sequences of sequences A, B, C and B' are shown in Table 3.

**Table 3: Example 3 nucleotide sequences**

| **Sequence name** | **Nucleotide sequence** | |
|---|---|---|
| Sequence A | ATATATATAT | SEQ ID 13 |
| Sequence B | CC | SEQ ID 14 |
| Sequence C | TATATATATA | SEQ ID 15 |
| Sequence D | GG | SEQ ID 16 |
| Sequence B' | CCCCCCCC | SEQ ID 17 |
| Sequence D' | GGGGGGGG | SEQ ID 18 |

The first SS oligonucleotide comprises the sequences in the following order: ⁵' - ABC - ³' (SEQ ID 19) and the second SS oligonucleotide comprises the sequences in the following order ^{3'} - CB' A - ⁵' (SEQ ID 20).

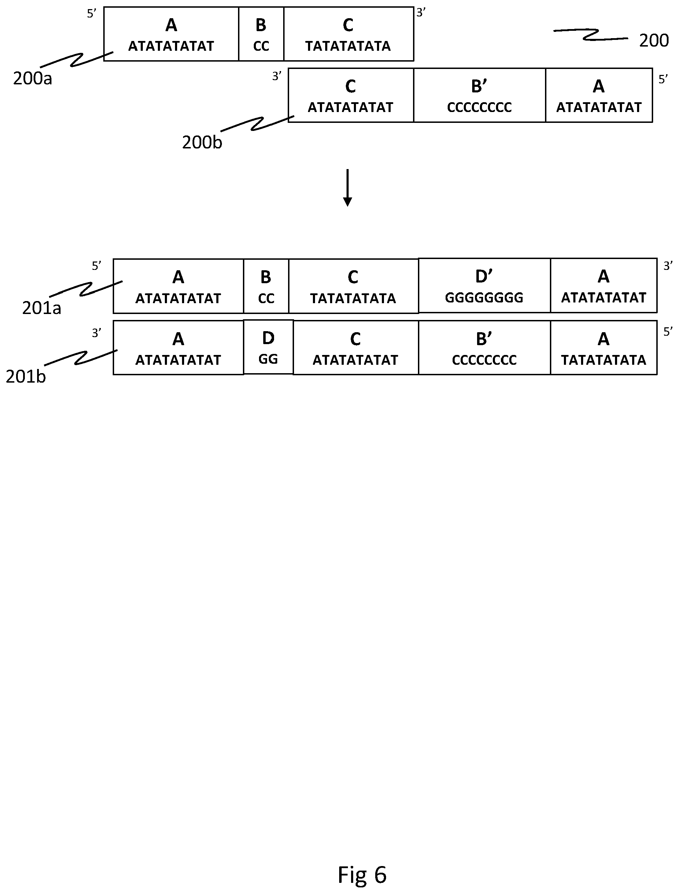

As in the previous examples, the A sequences are palindromic, self-complementary sequences and the C sequences are palindromic, self-complementary sequences. As shown in figure 6, a duplex 200 is formed by the hybridisation of sequence C in the first SS oligonucleotide 200a and second SS oligonucleotide 200b. The duplex 200 comprises a 5' overhang of sequences A and B on the first SS oligonucleotide 200a and sequences B' and A on the second SS oligonucleotide 200b.

During the enzymatic extension, complementary base pairing occurs to extend the first and second SS oligonucleotides at the 3' end. The first SS oligonucleotide is extended to include a sequence D' and A in an extended first SS oligonucleotide 201a. Sequence D' is complementary to sequence B'. The second SS oligonucleotide is extended to include sequence A and D in a second extended SS oligonucleotide 201b. Sequence D is complementary to sequence B. The nucleotide sequences of sequences D and D' can be found above in Table 3.

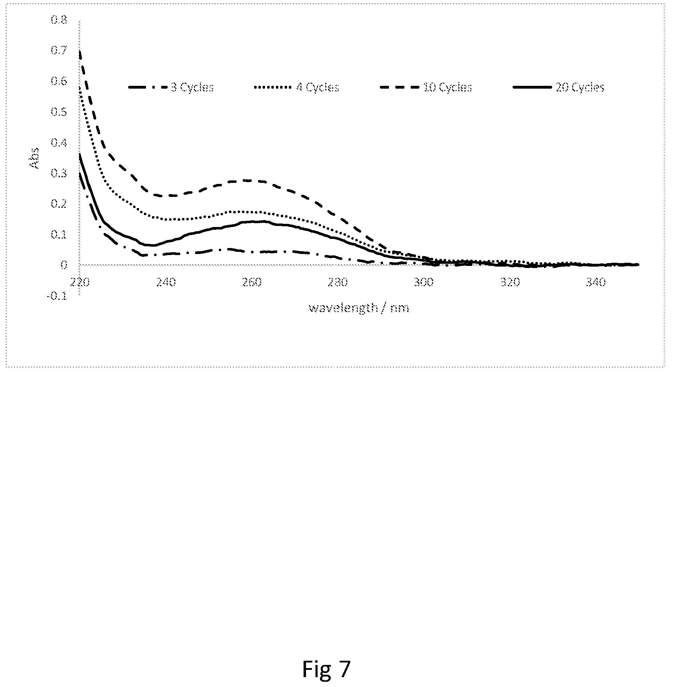

UV absorbance was measured as described in example 1. Figure 7 shows the UV absorbance data. Increased absorption of UV light at a wavelength of 260 nm indicates increased DNA concentration. As can be seen in figure 7, the DNA concentration in the PCR product increases with an increased number of PCR cycles, demonstrating generation of new DNA with increasing PCR cycle numbers.

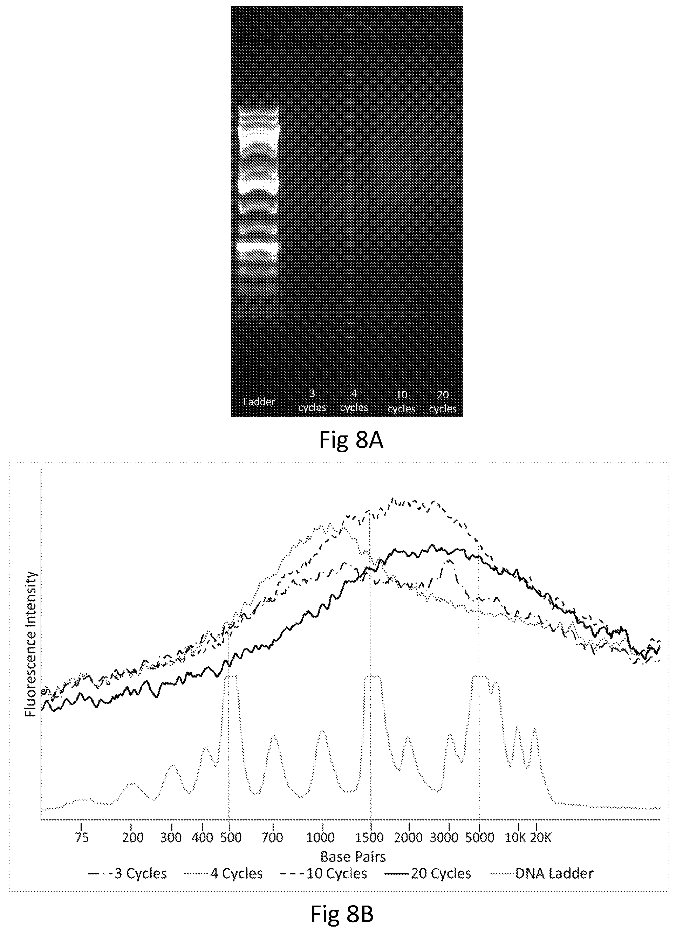

Figure 8A shows a visualisation of gel electrophoresis of the PCR products after 3, 4, 10 and 20 PCR cycles. The fluorescence intensity of the gel electrophoresis was measured and analysed using Image-J software, the results are shown in figure 8B. As can be seen in figure 8B, after 20 PCR cycles DNA products can be detected of between 1000 and 5000 base pairs in length.

### Materials and Methods

### Overlapping primer oligonucleotide preparation

The overlapping primer oligonucleotides (sequences shown in tables 1, 2 and 3) were prepared by adding the first and second SS oligonucleotides (10 µL of each SS oligonucleotide (100 µM)) to HEPES and potassium acetate DNA annealing buffer (480 µL, 1X) in an Eppendorf. HEPES and potassium acetate buffer is made from 10 mM HEPES, 100 mM KCI and 1mM EDTA. The solution was vortexed for a few seconds to ensure thorough mixing, and then heated to 95 °C for 10 minutes before being cooled slowly to room temperature (roughly 25 °C). When not in use, overlapping primer oligonucleotides were stored at -20°C in the freezer.

### DNA extension

Nanopure-H₂O (60 µL) was added to 10 µL each of dATP, dTTP, dCTP, dGTP (all 100 mM) to provide a 100 µL dNTP mix of 10 mM concentration. Primer-specific dNTP mixtures were prepared for each of the above examples by varying the volume of dNTPs added, depending on the ratio of bases present in the overlapping primer oligonucleotide. For example, the overlapping primer oligonucleotide used in example 1 ((AT)₅C₂(TA)₅ / (AT)₅C₂(TA)₅) has an A:T:C:G ratio of 5:5:1:1 and dNTPs were mixed at the same A:T:C:G ratio. For example, dATP (16.6 µL, 100 mM), dTTP (16.6 µL, 100 mM), dCTP (3.4 µL, 100 mM), and dGTP (3.4 µL, 100 mM) were added to nanopure-H₂O (60 µL) to make up the dNTP mix used in example 1.

### Heat-cool cycles

The extension reaction mixture was prepared in a thin-walled 200 µL Eppendorf by adding nanopure-H₂O (36 µL) to ThermoPol buffer solution (5 µL, 10X), overlapping primer oligonucleotide (5 µL, 2 µM), primer-specific dNTP mix (2.5 µL, 10mM), and MgSO₄ (1 µL, 100mM). The mixture was then vortexed for a few seconds before adding DeepVent DNA polymerase enzyme (0.5 µL, 1U). At 75 °C, one unit of DeepVent DNA polymerase enzyme can incorporate 10 nmol of dNTPs. To not destroy the polymerase enzyme, the mixture was mixed gently with a pipette tip instead of using a vortex. After 10 cycles, more primer-specific dNTP mix (2.5 µL, 10mM) was added, along with polymerase enzyme (0.5 µL, 1U). The Eppendorf was then placed back in the thermocycler on the same setting for a further 10 cycles (20 cycles in total). When not in use, reagents and products were stored at -20°C.

Each cycle consisted of:
1) Denaturing at 95 °C for 30 seconds.
2) Annealing at 55 °C for 30 seconds.
3) Elongation at 72 °C for 120 seconds.

After 10 cycles, the thermocycler cooled the mixture to 4 °C and held the mixture at this temperature for as long as required.

Different numbers of cycles were performed in each of the above examples. For 20 cycles, the procedure outlined above was followed. For 10 cycles, the first half of the procedure outlined above was followed (i.e., experiment was stopped after the first 10 cycles, thus not requiring the second addition of dNTP mix and polymerase enzyme).

### Purification of Extended DNA Product

For purification, the Monarch^{®} PCR and DNA Cleanup Kit was used. DNA binding buffer (100 µL) was added to the extended DNA solution and mixed with a pipette before transferring the whole sample to a thick-walled tube and column. The column was centrifuged at 13000 RPM for 1 minute, then the discarded buffer was disposed of in the aqueous waste. DNA wash buffer (200 µL) was added to the column, which was then centrifuged at 13000 RPM for 1 minute. This step was repeated, then all the wash solution was disposed of in the aqueous waste. The column was centrifuged again at 13000 RPM for 1 minute to ensure all excess liquid had been removed and the column was fully dry - any waste was disposed of in the aqueous waste. A 1.5 mL Eppendorf tube was heated to 65 °C in the heating block, into which the column (with DNA attached to the silica) was placed. This heating step was to increase yield. Elution buffer (20 µL) was added to the column in the heating block and was kept at 65 °C for 5 minutes. The sample was centrifuged for a final time at 13000 RPM for 1 minute. The purified extended DNA product was collected in the 1.5 mL Eppendorf tube. All binding/wash/elution buffers were stored at room temperature.

### UV/Vis Spectroscopy

UV/Vis spectra, overlapping primer oligonucleotide concentrations, and purity ratios were recorded using a Thermo Scientific^{™} NanoDrop^{™} One Microvolume-UV/Vis Spectrophotometer allowing analysis of 1 µL samples, using surface tension to hold the small volume of sample in place between two pedestals. Most measurements of samples and blanks were recorded using the dsDNA setting. Blanks were obtained using Monarch^{®} DNA Elution Buffer each time before performing measurements of each DNA sample. Between each measurement, a lint free wipe was used to clean the pedestals and limit any contamination between samples. An absorption band at 260 nm would confirm the presence of DNA.

### Agarose Gel Electrophoresis

Gels were electrophoresed at 100 V (400 mA) for 60 to 90 minutes. All gels were imaged using UviProMW1 software and a Uvitec Chemiluminescence fluorescence imaging box. All gels were analysed using ImageJ software, converting the data into graphs using Microsoft Excel.

### Example 4: inclusion of a restriction site

In another example of the present invention, a restriction site is included in the starting overlapping primer oligonucleotide. After extension the restriction site can be used to cut the nucleic acid to give two different length oligonucleotides. The nucleotide sequences of sequences A, B, C, D and E are shown in Table 4.

**Table 4: Example 4 nucleotide sequences**

| **Sequence name** | **Nucleotide sequence** | |
|---|---|---|
| Sequence A | 5'-TGGACTCTCTCA | SEQ ID 21 |
| Sequence B | 5'-GATATC | SEQ ID 22 |
| Sequence C | 5' -ATCGACT | SEQ ID 23 |
| Sequence D | **5'-AGTCGAT** | SEQ ID 24 |
| Sequence E | 5'-TGAGAGAGTCCA | SEQ ID 25 |

In this example:
A is complementary to E;
C is complementary to D; and
B is a self-complementary restriction site which splits into two portions and adds 3 bases to each end of the oligomers after restriction digest.

### After extension the product is long double-stranded 5'-[ABCB]ₙ-3' / 3'-[EBDB]ₙ-5'

Restriction enzyme treatment yields two double stranded oligomers of 18 and 13 bases, derived from the A/E complementary sequence and the C/D complementary sequence respectively:
18mer:
   5' - ATCTGGACTCTCTCAGAT -3' (A) (SEQ ID 26)
   3' - GATACCTGAGAGAGAGTCTA -5'(E)
13mer:
   5' - **ATCATCGACTGAT** -3' (C) (SEQ ID 27)
   3' **-GATTAGCTGACTA** -3' (D)

The overlapping primer oligonucleotides (sequences shown below) were prepared by adding the first and second ss oligonucleotides (10 µL of each ss oligonucleotide (100 µM)) to HEPES and potassium acetate DNA annealing buffer (480 µL, 1X) in an Eppendorf. HEPES and potassium acetate buffer is made from 10 mM HEPES, 100 mM KCI and 1 mM EDTA. The solution was vortexed for a few seconds to ensure thorough mixing, and then heated to 95 °C for 10 minutes before being cooled slowly to room temperature (roughly 25 °C). When not in use, overlapping primer oligonucleotides were stored at -20 °C in the freezer.
5' TGG ACT CTC TCA GAT ATC **ATC** GAC **T** 3'
3' **TAG CTG** ACT ATA GAC CTG AGA GAG T 5'

Nanopure-H₂O (60 µL) was added to 10 µL each of dATP, dTTP, dCTP, dGTP (all 100 mM) to provide a 100 µL dNTP mix of 10 mM concentration.

The extension reaction mixture was prepared in a thin-walled 200 µL Eppendorf by adding nanopure-H₂O (36 µL) to ThermoPol buffer solution (5 µL, 10X), overlapping primer oligonucleotide (5 µL, 2 µM), primer-specific dNTP mix (2.5 µL, 10 mM), and MgSO₄ (1 µL, 100 mM). The mixture was then vortexed for a few seconds before adding DeepVent DNA polymerase enzyme (0.5 µL, 1U). At 75 °C, one unit of DeepVent (exo-) DNA polymerase enzyme can incorporate 10 nmol of dNTPs. To not destroy the polymerase enzyme, the mixture was mixed gently with a pipette tip instead of using a vortex. After 10 cycles, more primer-specific dNTP mix (2.5 µL, 10mM) was added, along with polymerase enzyme (0.5 µL, 1U). The Eppendorf was then placed back in the thermocycler on the same setting for a further 10 cycles (20 cycles in total). When not in use, reagents and products were stored at -20 °C.

Each cycle consisted of:
1) Denaturing at 95 °C for 30 seconds.
2) Annealing at 55 °C for 30 seconds.
3) Elongation at 72 °C for 120 seconds.

After 20 cycles, the thermocycler cooled the mixture to 4 °C and held the mixture at this temperature for as long as required.

For purification, the Monarch^{®} PCR and DNA Cleanup Kit was used. DNA binding buffer (100 µL) was added to the extended DNA solution and mixed with a pipette before transferring the whole sample to a thick-walled tube and column. The column was centrifuged at 13000 RPM for 1 minute, then the discarded buffer was disposed of in the aqueous waste. DNA wash buffer (200 µL) was added to the column, which was then centrifuged at 13000 RPM for 1 minute. This step was repeated, then all the wash solution was disposed of in the aqueous waste. The column was centrifuged again at 13000 RPM for 1 minute to ensure all excess liquid had been removed and the column was fully dry - any waste was disposed of in the aqueous waste. A 1.5 mL Eppendorf tube was heated to 60 °C in the heating block, into which the column (with DNA attached to the silica) was placed. This heating step was to increase yield. Elution buffer (20 µL) was added to the column in the heating block and was kept at 65 °C for 5 minutes. The sample was centrifuged for a final time at 13000 RPM for 1 minute. The purified extended DNA product was collected in the 1.5 mL Eppendorf tube. All binding/wash/elution buffers were stored at room temperature.

### UV/Vis Spectroscopy

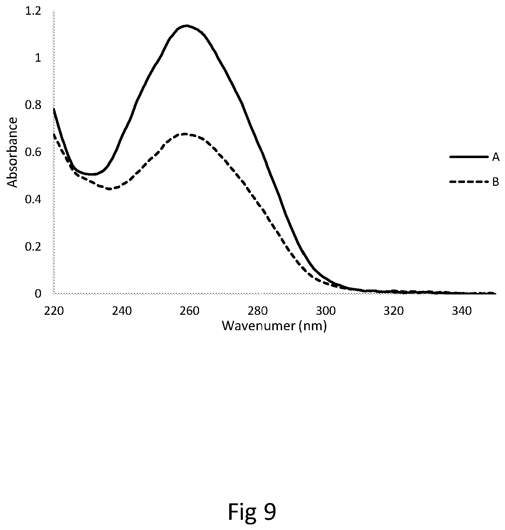

UV/Vis spectra, as shown in Figure 9, overlapping primer oligonucleotide concentrations, and purity ratios were recorded using a Thermo Scientific^{™} NanoDrop^{™} One Microvolume-UV/Vis Spectrophotometer allowing analysis of 1 µL samples, using surface tension to hold the small volume of sample in place between two pedestals. Most measurements of samples and blanks were recorded using the dsDNA setting. Blanks were obtained using Monarch^{®} DNA Elution Buffer each time before performing measurements of each DNA sample. Between each measurement, a lint free wipe was used to clean the pedestals and limit any contamination between samples. An absorption band at 260 nm confirms the presence of DNA.

### Agarose Gel Electrophoresis

Gels were electrophoresed at 100 V (400 mA) for 60 to 90 minutes. All gels were imaged using UviProMW1 software and a Uvitec Chemiluminescence fluorescence imaging box. All gels were analysed using ImageJ software, converting the data into graphs using Microsoft Excel.

### Digestion

Reaction mixture was made up using the extended dsDNA product, rCutSmart Buffer and EcoRV HF in the concentrations and volumes in table 5. This reaction mixture was heated to 37 °C for 3 hours before purple loading dye was added to deactivate the enzyme.

**Table 5 - restriction enzyme digest reaction components**

| | **Initial concentration** | **Volume (µL)** | **Final concentration** |
|---|---|---|---|
| DNA | 57 ng/µL | 19 | 20 ng/µL |
| Water | | 25 | |
| rCutSmart Buffer | 10X | 5 | 1X |
| EcoRV-HF | | 0.4 | 40 units |

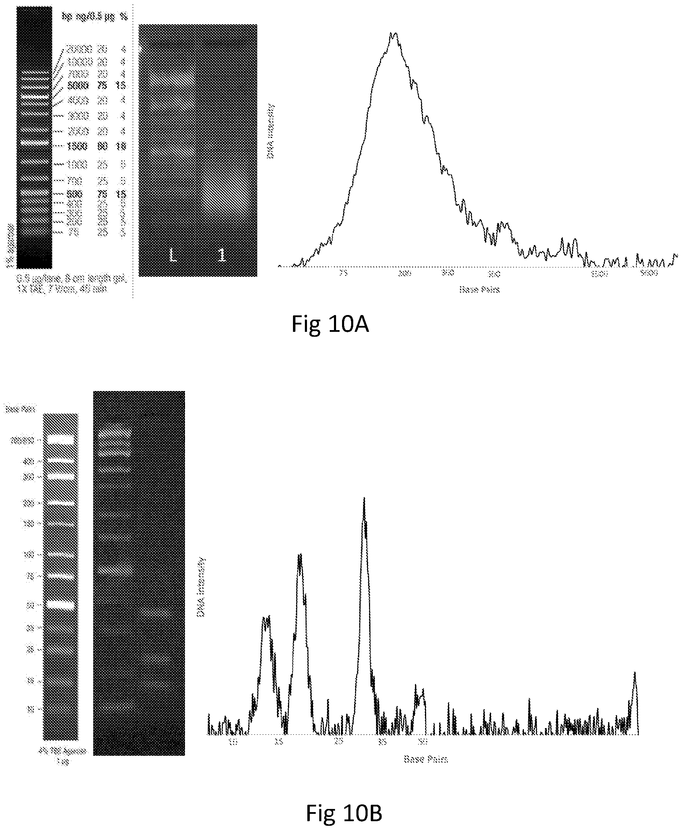

A 4% MetaPhor agarose gel was used to run the digestion product alongside a low range ladder and the gel electrophoresis was analysed using Image-J as shown in Figure 10.

Figure 10 (A) shows a visualisation of gel electrophoresis of the extended DNA against the Gene Ruler 1kb plus DNA ladder, the fluorescence intensity of the gel electrophoresis was measured and analysed using Image-J software, the results show extension products of 75-300 base pairs.

Figure 10 (B) shows a visualisation of gel electrophoresis of the digested products against a low range ladder, the fluorescence intensity of the gel electrophoresis was measured and analysed using Image-J software, the results are shown. As can be seen, after digestion, DNA bands of 31 bases (if only chopped at every other restriction site), 18 bases and 13 bases can be detected which correspond to the two products (A/E and C/D) as expected after digestion either side of the restriction sites.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims are generally intended as "open" terms (e.g., the term "including" or "comprising" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (e.g., the bare recitation of "two recitations," without other modifiers, means at least two recitations, or two or more recitations).

It will be appreciated that various embodiments of the present disclosure have been described herein for purposes of illustration.

## Claims

1. A method for extending the length of a nucleic acid, the method comprising the steps of;
i) providing at least one overlapping primer oligonucleotide, said overlapping primer oligonucleotide comprising a first single-stranded oligonucleotide and a second single-stranded oligonucleotide, each of the first and second single-stranded oligonucleotides comprising at least a first, second and third sequence, said third sequence being located at the 3' end of each single-stranded oligonucleotide and said second sequence being located between the first and third sequences; wherein the first sequences and the third sequences of the first single-stranded oligonucleotide and the second single-stranded oligonucleotide are self-complementary, palindromic sequences;
wherein the third sequence of the first single-stranded oligonucleotide hybridises to the third sequence of the second single-stranded oligonucleotide to provide an overhanging region at the 5' end of at least one of the first or second single-stranded oligonucleotides;
and wherein the second sequence on the first single-stranded oligonucleotide differs from the second sequence on the second single-stranded oligonucleotide;
(ii) enzymatically extending both the first and second single-stranded oligonucleotides to provide a duplex.

2. A method for extending the length of a nucleic acid according to claim 1, wherein a fourth sequence is incorporated into the first and second single-stranded oligonucleotides during step (ii), said fourth sequence on the first single-stranded oligonucleotide being complementary to the second sequence on the second single-stranded oligonucleotide and said fourth sequence on the second single-stranded oligonucleotide being complementary to the second sequence on the first single-stranded oligonucleotide.

3. A method for extending the length of a nucleic acid according to any previous claim, wherein the method further comprises the steps:
(iii) denaturing the duplex to provide a first and second single-stranded polynucleotide;
(iv) annealing the first and second single-stranded polynucleotides such that overhanging region is provided at the 5' end of at least one of the first or second single-stranded polynucleotides to permit polynucleotide duplex formation between the first and second single-stranded polynucleotides;
(v) enzymatically extending both the first and second single-stranded polynucleotides to provide a duplex.

4. A method for extending the length of a nucleic acid according to claim 3, wherein steps (iii) to (v) are repeated to increase the length of the nucleic acid sequence.

5. A method for extending the length of a nucleic acid according to any previous claim, wherein the overlapping primer oligonucleotide is immobilised on a surface, and, preferably, wherein the overlapping primer oligonucleotide is immobilised on a surface by the 5' end of one of the first or second single-stranded polynucleotides; optionally wherein the surface comprises glass, silica, gold, graphene, graphene oxide, epoxy, plastic, metal, gel matrix, template stripped metals or composites thereof.

6. A method for extending the length of a nucleic acid according to claim 5, wherein the overlapping primer oligonucleotide is immobilised to the surface by covalent or non-covalent bonding;
and/or wherein the overlapping primer oligonucleotide is immobilised to the surface by a linker wherein, preferably, the linker comprises a silane linker molecule, a biotin-streptavidin complex, a thiol-Au linker, covalent Si-C bonds to silicon, covalent Si-O bonds to silicon, covalent Si-N bonds to silicon, a nanoparticle linker, or a dynamic covalent bond;
and/or wherein the overlapping primer oligonucleotide is immobilised to a chemically modified region of the surface.

7. An overlapping primer oligonucleotide for extending the length of repeating sequences of nucleic acid, said overlapping primer oligonucleotide comprising two partially complementary single-stranded oligonucleotides, each single-stranded oligonucleotide comprising at least a first, second and third sequence wherein the third sequence is located at the 3' end of the first and second single-stranded oligonucleotide; the first sequences and the third sequences are palindromic, self-complementary sequences; and the second sequence is located in between the first and third sequences; wherein the third sequence of the first single-stranded oligonucleotide hybridises to the third sequence of the second single-stranded oligonucleotide to provide an overhanging region at the 5' end of at least one of the first or second single-stranded oligonucleotides;
and wherein the second sequence on the first single-stranded oligonucleotide differs from the second sequence on the second single-stranded oligonucleotide and/or wherein the second sequence on the first single-stranded oligonucleotide is not complementary to the second sequence on the second single-stranded oligonucleotide.

8. A method for extending the length of a nucleic acid according to any of claims 1 to 6 or an overlapping primer oligonucleotide according to claim 7 , wherein the first and third sequences are each at least 6 bases in length and are preferably at least 8 bases in length.

9. A method for extending the length of a nucleic acid according to any of claims 1 to 6 or 8, or an overlapping primer oligonucleotide according to any of claims 7 to 8, wherein the second sequence is at least 2 bases in length.

10. A method for extending the length of a nucleic acid according to any of claims 1 to 6, 8 or 9 or an overlapping primer oligonucleotide according to any of claims 7 to 9, wherein at least one of the first and third sequences comprises at least one functional site and wherein the at least one functional site may be selected from the following: restriction enzyme site, capping site, sequence specific drug binding site, enzyme inhibitor site.

11. A method for extending the length of a nucleic acid according to any of claims 1 to 6, or 8 to 10, or an overlapping primer oligonucleotide according to any of claims 7 to 10, wherein at least one of the first and third sequences comprise tandem repeats, optionally wherein the tandem repeat is a dinucleotide tandem repeat.

12. A method for extending the length of a nucleic acid according to any of claims 1 to 6, or 8 to 11, or an overlapping primer oligonucleotide according to any of claims 7 to 11, wherein the second sequence comprises at least one modification selected from the following: modified nucleotides, artificial bases, loop structures.

13. A method for extending the length of a nucleic acid, or an overlapping primer oligonucleotide according to claim 12, wherein the modified nucleotides are alkyne, azide or phosphorothioate modified nucleotides, optionally, these may include: 5-Br-dUTP, 7-deaza-7-I-dATP, 6-S-dGTP,5-I-dCTP, 5-(octadiynyl)-dCTP; and/or wherein, the modified nucleotides comprise a linker which attaches the modification to the nucleotide

14. A method for extending the length of a nucleic acid, or an overlapping primer oligonucleotide according to claim 12, wherein the loop structure is a G quadruplex region or a C-motif or intercalated-motif DNA.

15. A method for extending the length of a nucleic acid according to any of claims 1 to 6 or 8 to 14, or an overlapping primer oligonucleotide according to any of claims 7 to 14, wherein the overlapping primer oligonucleotide is a DNA primer oligonucleotide or an RNA primer oligonucleotide.

## Patentansprüche

1. Verfahren zum Verlängern der Länge einer Nukleinsäure, wobei das Verfahren die folgenden Schritte umfasst;
(i) Bereitstellen mindestens eines überlappenden Primer-Oligonukleotids, wobei das überlappende Primer-Oligonukleotid ein erstes einzelsträngiges Oligonukleotid und ein zweites einzelsträngiges Oligonukleotid umfasst, wobei jedes der ersten und zweiten einzelsträngigen Oligonukleotide mindestens eine erste, zweite und dritte Sequenz umfasst, wobei sich die dritte Sequenz am 3'-Ende jedes einzelsträngigen Oligonukleotids befindet und sich die zweite Sequenz zwischen der ersten und der dritten Sequenz befindet; wobei die ersten Sequenzen und die dritten Sequenzen des ersten einzelsträngigen Oligonukleotids und des zweiten einzelsträngigen Oligonukleotids selbstkomplementäre, palindromische Sequenzen sind;
wobei die dritte Sequenz des ersten einzelsträngigen Oligonukleotids mit der dritten Sequenz des zweiten einzelsträngigen Oligonukleotids hybridisiert, um einen überhängenden Bereich am 5'-Ende mindestens eines der ersten oder zweiten einzelsträngigen Oligonukleotide bereitzustellen;
und wobei sich die zweite Sequenz auf dem ersten einzelsträngigen Oligonukleotid von der zweiten Sequenz auf dem zweiten einzelsträngigen Oligonukleotid unterscheidet;
(ii) enzymatisches Verlängern sowohl des ersten als auch des zweiten einzelsträngigen Oligonukleotids, um ein Duplex bereitzustellen.

2. Verfahren zum Verlängern der Länge einer Nukleinsäure nach Anspruch 1, wobei eine vierte Sequenz während Schritt (ii) in das erste und zweite einzelsträngige Oligonukleotid eingebaut wird, wobei die vierte Sequenz auf dem ersten einzelsträngigen Oligonukleotid komplementär zur zweiten Sequenz auf dem zweiten einzelsträngigen Oligonukleotid ist und die vierte Sequenz auf dem zweiten einzelsträngigen Oligonukleotid komplementär zur zweiten Sequenz auf dem ersten einzelsträngigen Oligonukleotid ist.

3. Verfahren zum Verlängern der Länge einer Nukleinsäure nach einem der vorhergehenden Ansprüche, wobei das Verfahren ferner die folgenden Schritte umfasst:
(iii) Denaturieren des Duplexes, um ein erstes und ein zweites einzelsträngiges Polynukleotid bereitzustellen;
(iv) Anlagern des ersten und zweiten einzelsträngigen Polynukleotids, so dass ein überhängender Bereich am 5'-Ende mindestens eines der ersten oder zweiten einzelsträngigen Polynukleotide bereitgestellt wird, um die Bildung eines Polynukleotid-Duplexes zwischen dem ersten und zweiten einzelsträngigen Polynukleotid zu ermöglichen;
(v) enzymatisches Verlängern sowohl des ersten als auch des zweiten einzelsträngigen Polynukleotids, um ein Duplex bereitzustellen.

4. Verfahren zum Verlängern der Länge einer Nukleinsäure nach Anspruch 3, wobei die Schritte (iii) bis (v) wiederholt werden, um die Länge der Nukleinsäuresequenz zu erhöhen.

5. Verfahren zum Verlängern der Länge einer Nukleinsäure nach einem der vorhergehenden Ansprüche, wobei das überlappende Primer-Oligonukleotid auf einer Oberfläche immobilisiert ist und wobei das überlappende Primer-Oligonukleotid vorzugsweise durch das 5'-Ende eines der ersten oder zweiten einzelsträngigen Polynukleotide auf einer Oberfläche immobilisiert ist; wobei die Oberfläche optional Glas, Siliziumdioxid, Gold, Graphen, Graphenoxid, Epoxid, Kunststoff, Metall, Gelmatrix, templatabgetragene Metalle oder Verbundstoffe davon umfasst.

6. Verfahren zum Verlängern der Länge einer Nukleinsäure nach Anspruch 5, wobei das überlappende Primer-Oligonukleotid durch kovalente oder nichtkovalente Bindung an der Oberfläche immobilisiert ist;
und/oder wobei das überlappende Primer-Oligonukleotid durch einen Linker an der Oberfläche immobilisiert ist, wobei der Linker vorzugsweise ein Silan-Linkermolekül, einen Biotin-Streptavidin-Komplex, einen Thiol-Au-Linker, kovalente Si-C-Bindungen an Silizium, kovalente Si-O-Bindungen an Silizium, kovalente Si-N-Bindungen an Silizium, einen Nanopartikel-Linker oder eine dynamische kovalente Bindung umfasst;
und/oder wobei das überlappende Primer-Oligonukleotid an einem chemisch modifizierten Bereich der Oberfläche immobilisiert ist.

7. Überlappendes Primer-Oligonukleotid zum Verlängern der Länge sich wiederholender Sequenzen von Nukleinsäure, wobei das überlappende Primer-Oligonukleotid zwei teilweise komplementäre einzelsträngige Oligonukleotide umfasst, wobei jedes einzelsträngige Oligonukleotid mindestens eine erste, zweite und dritte Sequenz umfasst, wobei sich die dritte Sequenz am 3'-Ende des ersten und zweiten einzelsträngigen Oligonukleotids befindet; wobei die ersten Sequenzen und die dritten Sequenzen palindromische, selbstkomplementäre Sequenzen sind; und wobei sich die zweite Sequenz zwischen der ersten und der dritten Sequenz befindet; wobei die dritte Sequenz des ersten einzelsträngigen Oligonukleotids mit der dritten Sequenz des zweiten einzelsträngigen Oligonukleotids hybridisiert, um einen überhängenden Bereich am 5'-Ende mindestens eines der ersten oder zweiten einzelsträngigen Oligonukleotide bereitzustellen;
und wobei sich die zweite Sequenz auf dem ersten einzelsträngigen Oligonukleotid von der zweiten Sequenz auf dem zweiten einzelsträngigen Oligonukleotid unterscheidet und/oder wobei die zweite Sequenz auf dem ersten einzelsträngigen Oligonukleotid nicht komplementär zur zweiten Sequenz auf dem zweiten einzelsträngigen Oligonukleotid ist.

8. Verfahren zum Verlängern der Länge einer Nukleinsäure nach einem der Ansprüche 1 bis 6 oder überlappendes Primer-Oligonukleotid nach Anspruch 7, wobei die erste und dritte Sequenz jeweils mindestens 6 Basen lang sind und vorzugsweise mindestens 8 Basen lang sind.

9. Verfahren zum Verlängern der Länge einer Nukleinsäure nach einem der Ansprüche 1 bis 6 oder 8 oder überlappendes Primer-Oligonukleotid nach einem der Ansprüche 7 bis 8, wobei die zweite Sequenz mindestens 2 Basen lang ist.

10. Verfahren zum Verlängern der Länge einer Nukleinsäure nach einem der Ansprüche 1 bis 6, 8 oder 9 oder überlappendes Primer-Oligonukleotid nach einem der Ansprüche 7 bis 9, wobei mindestens eine der ersten und dritten Sequenzen mindestens eine funktionelle Stelle umfasst und wobei die mindestens eine funktionelle Stelle aus den folgenden ausgewählt sein kann: Restriktionsenzymstelle, Capping-Stelle, sequenzspezifische Wirkstoffbindungsstelle, Enzymhemmerstelle.

11. Verfahren zum Verlängern der Länge einer Nukleinsäure nach einem der Ansprüche 1 bis 6 oder 8 bis 10 oder überlappendes Primer-Oligonukleotid nach einem der Ansprüche 7 bis 10, wobei mindestens eine der ersten und dritten Sequenzen Tandem-Wiederholungen umfasst, wobei die Tandem-Wiederholung optional eine Dinukleotid-Tandem-Wiederholung ist.

12. Verfahren zum Verlängern der Länge einer Nukleinsäure nach einem der Ansprüche 1 bis 6 oder 8 bis 11 oder überlappendes Primer-Oligonukleotid nach einem der Ansprüche 7 bis 11, wobei die zweite Sequenz mindestens eine Modifikation umfasst, die aus den folgenden ausgewählt ist: modifizierte Nukleotide, künstliche Basen, Schleifenstrukturen.

13. Verfahren zum Verlängern der Länge einer Nukleinsäure oder überlappendes Primer-Oligonukleotid nach Anspruch 12, wobei die modifizierten Nukleotide Alkin-, Azid- oder Phosphorthioat-modifizierte Nukleotide sind, die optional Folgendes einschließen können: 5-Br-dUTP, 7-Deaza-7-l-dATP, 6-S-dGTP, 5-l-dCTP, 5-(Octadiynyl)-dCTP; und/oder wobei die modifizierten Nukleotide einen Linker umfassen, der die Modifikation an das Nukleotid bindet.

14. Verfahren zum Verlängern der Länge einer Nukleinsäure oder überlappendes Primer-Oligonukleotid nach Anspruch 12, wobei die Schleifenstruktur eine G-Quadruplex-Region oder eine C-Motiv- oder interkalierte Motiv-DNA ist.

15. Verfahren zum Verlängern der Länge einer Nukleinsäure nach einem der Ansprüche 1 bis 6 oder 8 bis 14 oder überlappendes Primer-Oligonukleotid nach einem der Ansprüche 7 bis 14, wobei das überlappende Primer-Oligonukleotid ein DNA-Primer-Oligonukleotid oder ein RNA-Primer-Oligonukleotid ist.

## Revendications

1. Procédé permettant d'étendre la longueur d'un acide nucléique, le procédé comprenant les étapes consistant à ;
(i) fournir au moins un oligonucléotide amorce chevauchant, ledit oligonucléotide amorce chevauchant comprenant un premier oligonucléotide simple brin et un second oligonucléotide simple brin, chacun des premier et second oligonucléotides simple brin comprenant au moins une première, une deuxième et une troisième séquence, ladite troisième séquence étant située au niveau de l'extrémité 3' de chaque oligonucléotide simple brin et ladite deuxième séquence étant située entre les première et troisième séquences ; dans lequel les premières séquences et les troisièmes séquences du premier oligonucléotide simple brin et du second oligonucléotide simple brin sont des séquences palindromiques auto-complémentaires ;
dans lequel la troisième séquence du premier oligonucléotide simple brin s'hybride à la troisième séquence du second oligonucléotide simple brin pour fournir une région en surplomb au niveau de l'extrémité 5' d'au moins l'un parmi le premier et le second oligonucléotides simple brin ;
et dans lequel la deuxième séquence sur le premier oligonucléotide simple brin diffère de la deuxième séquence sur le second oligonucléotide simple brin ;
(ii) étendre par voie enzymatique les premier et second oligonucléotides simple brin pour former un duplex.

2. Procédé permettant d'étendre la longueur d'un acide nucléique selon la revendication 1, dans lequel une quatrième séquence est incorporée dans les premier et second oligonucléotides simple brin au cours de l'étape (ii), ladite quatrième séquence sur le premier oligonucléotide simple brin étant complémentaire de la deuxième séquence sur le second oligonucléotide simple brin et ladite quatrième séquence sur le second oligonucléotide simple brin étant complémentaire de la deuxième séquence sur le premier oligonucléotide simple brin.

3. Procédé permettant d'étendre la longueur d'un acide nucléique selon l'une quelconque revendication précédente, dans lequel le procédé comprend en outre les étapes suivantes :
(iii) la dénaturation du duplex pour fournir un premier et un second polynucléotide simple brin ;
(iv) la recuisson des premier et second polynucléotides simple brin de telle sorte qu'une région en surplomb est prévue au niveau de l'extrémité 5' d'au moins l'un parmi les premier ou second polynucléotides simple brin pour permettre la formation d'un duplex polynucléotidique entre les premier et second polynucléotides simple brin ;
(v) l'extension par voie enzymatique des premier et second polynucléotides simple brin pour fournir un duplex.

4. Procédé permettant d'étendre la longueur d'un acide nucléique selon la revendication 3, dans lequel les étapes (iii) à (v) sont répétées pour augmenter la longueur de la séquence d'acide nucléique.

5. Procédé permettant d'étendre la longueur d'un acide nucléique selon l'une quelconque revendication précédente, dans lequel l'oligonucléotide amorce chevauchant est immobilisé sur une surface, et, de préférence, dans lequel l'oligonucléotide amorce chevauchant est immobilisé sur une surface par l'extrémité 5' de l'un des premier ou second polynucléotides simple brin ; éventuellement dans lequel la surface comprend du verre, de la silice, de l'or, du graphène, de l'oxyde de graphène, de l'époxy, du plastique, du métal, une matrice de gel, des métaux dépourvus de gabarit ou des composites de ceux-ci.

6. Procédé permettant d'étendre la longueur d'un acide nucléique selon la revendication 5, dans lequel l'oligonucléotide amorce chevauchant est immobilisé à la surface par liaison covalente ou non covalente ;
et/ou dans lequel l'oligonucléotide amorce chevauchant est immobilisé à la surface par un agent de liaison dans lequel, de préférence, l'agent de liaison comprend une molécule de liaison silane, un complexe biotine-streptavidine, un agent de liaison thiol-Au, des liaisons covalentes Si-C au silicium, des liaisons covalentes Si-O au silicium, des liaisons covalentes Si-N au silicium, un agent de liaison nanoparticule ou une liaison covalente dynamique ;
et/ou dans lequel l'oligonucléotide amorce chevauchant est immobilisé sur une région chimiquement modifiée de la surface.

7. Oligonucléotide amorce chevauchant permettant d'étendre la longueur des séquences répétitives d'acide nucléique, ledit oligonucléotide amorce chevauchant comprenant deux oligonucléotides simple brin partiellement complémentaires, chaque oligonucléotide simple brin comprenant au moins une première, une deuxième et une troisième séquence dans lequel la troisième séquence est située au niveau de l'extrémité 3' des premier et second oligonucléotides simple brin ; les premières séquences et les troisièmes séquences sont des séquences palindromiques, auto-complémentaires ; et la deuxième séquence est située entre les premières et troisièmes séquences ; dans lequel la troisième séquence du premier oligonucléotide simple brin s'hybride à la troisième séquence du second oligonucléotide simple brin pour fournir une région en surplomb au niveau de l'extrémité 5' d'au moins l'un parmi les premier ou second oligonucléotides simple brin ;
et dans lequel la deuxième séquence sur le premier oligonucléotide simple brin diffère de la deuxième séquence sur le second oligonucléotide simple brin et/ou dans lequel la deuxième séquence sur le premier oligonucléotide simple brin n'est pas complémentaire de la deuxième séquence sur le second oligonucléotide simple brin.

8. Procédé permettant d'étendre la longueur d'un acide nucléique selon l'une quelconque des revendications 1 à 6 ou d'un oligonucléotide amorce chevauchant selon la revendication 7, dans lequel les première et troisième séquences ont chacune une longueur d'au moins 6 bases et ont de préférence une longueur d'au moins 8 bases.

9. Procédé permettant d'étendre la longueur d'un acide nucléique selon l'une quelconque des revendications 1 à 6 ou 8, ou d'un oligonucléotide amorce chevauchant selon l'une quelconque des revendications 7 à 8, dans lequel la deuxième séquence a une longueur d'au moins 2 bases.

10. Procédé permettant d'étendre la longueur d'un acide nucléique selon l'une quelconque des revendications 1 à 6, 8 ou 9 ou d'un oligonucléotide amorce chevauchant selon l'une quelconque des revendications 7 à 9, dans lequel au moins l'une parmi les première et troisième séquences comprend au moins un site fonctionnel et dans lequel l'au moins un site fonctionnel peut être sélectionné parmi les suivants : un site d'enzyme de restriction, un site de coiffage, un site de liaison de médicament spécifique à une séquence, un site inhibiteur d'enzyme.

11. Procédé permettant d'étendre la longueur d'un acide nucléique selon l'une quelconque des revendications 1 à 6, ou 8 à 10, ou d'un oligonucléotide amorce chevauchant selon l'une quelconque des revendications 7 à 10, dans lequel au moins l'une parmi les première et troisième séquences comprend des répétitions en tandem, éventuellement dans lequel la répétition en tandem est une répétition en tandem dinucléotidique.

12. Procédé permettant d'étendre la longueur d'un acide nucléique selon l'une quelconque des revendications 1 à 6, ou 8 à 11, ou d'un oligonucléotide amorce chevauchant selon l'une quelconque des revendications 7 à 11, dans lequel la deuxième séquence comprend au moins une modification sélectionnée parmi les suivantes : des nucléotides modifiés, des bases artificielles, des structures en boucle.

13. Procédé permettant d'étendre la longueur d'un acide nucléique, ou d'un oligonucléotide amorce chevauchant selon la revendication 12, dans lequel les nucléotides modifiés sont des nucléotides modifiés par un alcyne, un azide ou un phosphorothioate, éventuellement, ceux-ci peuvent comprendre : 5-Br-dUTP, 7-déaza-7-l-dATP, 6-S-dGTP, 5-l-dCTP, 5-(octadiynyl)-dCTP ; et/ou dans lequel les nucléotides modifiés comprennent un agent de liaison qui fixe la modification au nucléotide.

14. Procédé permettant d'étendre la longueur d'un acide nucléique, ou d'un oligonucléotide amorce chevauchant selon la revendication 12, dans lequel la structure en boucle est une région quadruplex G ou un ADN à motif C ou à motif intercalé.

15. Procédé permettant d'étendre la longueur d'un acide nucléique selon l'une quelconque des revendications 1 à 6 ou 8 à 14, ou d'un oligonucléotide amorce chevauchant selon l'une quelconque des revendications 7 à 14, dans lequel l'oligonucléotide amorce chevauchant est un oligonucléotide amorce d'ADN ou un oligonucléotide amorce d'ARN.
